(19) **Europäisches Patentamt**
**European Patent Office**
**Office européen des brevets**

(11) **EP 4 501 464 B1**

(12) **EUROPEAN PATENT SPECIFICATION**

(45) Date of publication and mention
of the grant of the patent:
**29.10.2025 Bulletin 2025/44**

(21) Application number: **23780783.9**

(22) Date of filing: **29.03.2023**

(51) International Patent Classification (IPC):
*B05B 13/00* (2006.01)    *H05H 1/24* (2006.01)
*A61N 1/44* (2006.01)    *B01J 19/08* (2006.01)
*C02F 1/48* (2023.01)

(52) Cooperative Patent Classification (CPC):
**A61N 1/44; B05B 5/03; B05B 5/0535;**
**B05B 5/1608; H05H 1/245;** B05B 5/0536

(86) International application number:
**PCT/JP2023/013003**

(87) International publication number:
**WO 2023/190775 (05.10.2023 Gazette 2023/40)**

(54) **PLASMA NANO MIST GENERATION DEVICE**

PLASMANANONEBELERZEUGUNGSVORRICHTUNG

DISPOSITIF DE GÉNÉRATION DE NANO-BROUILLARD DE PLASMA

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **30.03.2022 JP 2022057334**

(43) Date of publication of application:
**05.02.2025 Bulletin 2025/06**

(73) Proprietor: **National University Corporation**
**Tokyo University Of Agriculture and Technology**
**Fuchu-shi**
**Tokyo 183-8538 (JP)**

(72) Inventors:
• **YOSHINO, Daisuke**
  **Fuchu-shi, Tokyo 183-8538 (JP)**
• **WATANABE, Ryosuke**
  **Fuchu-shi, Tokyo 183-8538 (JP)**
• **TANAKA, Shiori**
  **Fuchu-shi, Tokyo 183-8538 (JP)**

(74) Representative: **V.O.**
**P.O. Box 87930**
**2508 DH Den Haag (NL)**

(56) References cited:
**EP-A1- 3 496 515**    **JP-A- 2011 229 571**
**JP-A- 2013 211 153**    **JP-A- 2018 504 451**
**JP-A- 2019 058 862**    **KR-B1- 101 500 155**
**US-A1- 2022 095 444**

## Description

Technical Field

The present disclosure relates to a plasma nano mist generation device.

Background Art

Plasma under atmospheric pressure generates electric fields, optical radiation, heat, and reactive chemical species, and thus is an important technology supporting many innovations in the industry. In recent years, applications of plasma systems in the fields of medicine and biology have been sought as effective tools for wound healing (see, for example, WO 2011/071182) and cancer treatment (see, for example, "Masaaki Nagatsu, Enbo Yang, Han Cho, Akikazu Sakudo, Kazuyoshi Ikuta, Bio- and Medical-Application of Plasma-Functionalized Nanoparticles, Journal of the Surface Science Society of Japan, Vol. 34, No. 10, pp. 535-540, 2013.").

These biomedical applications are mainly based on direct action of plasma-derived physical and chemical stimuli (ultraviolet rays, active oxygen, nitrogen species, etc.) on an object. Unless the affected part is exposed like a wound, application of plasma directly to the lesion could give a large surgical burden on the patient.

However, biomedical technology that indirectly uses the effect of plasma has not been studied much. For example, if a form operation of a drug or a drug solution by plasma can be applied to promote transdermal absorption of the drug or the drug solution, new development of a medical technology for reducing a load on the whole body can be expected.

In order to promote transdermal absorption of a drug, formation of nanoparticles of the drug is important. Nanotechnology-based delivery systems allow a drug to pass through the skin barrier and to reach the target site in a stable condition, and maintain efficacy of the drug for an extended period of time. Plasma-generating nanoparticles (see, for example, "Masaaki Nagatsu, Enbo Yang, Han Cho, Akikazu Sakudo, Kazuyoshi Ikuta, Bio- and Medical-Application of Plasma-Functionalized Nanoparticles, Journal of the Surface Science Society of Japan, Vol. 34, No. 10, pp. 535-540, 2013." and "Michael, P., Lam, Y.T., Filipe, E.C. et al. Plasma polymerized nanoparticles effectively deliver dual siRNA and drug therapy in vivo. Sci Rep 10, 12836 (2020). https://doi.org/10.1038/s41598-020-69591-x.") can be used in such delivery systems.

However, there is a disadvantage of requiring complex chemical processes. Further development of transdermal drug delivery can be expected if a nano-sized particulate assembly can be directly generated by a simple process from an aqueous solution or an oily solution in which a drug is dissolved.

As a technique for making a solution into nanoparticles (generating nano-sized mist) using an electrical effect, an electrostatic atomization method is known (see, for example, WO 2011/071182 and Japanese Patent Application Laid-Open No. 2019-058862). In addition, there is also known a technique for improving the transdermal absorption effect by applying an atmospheric pressure plasma treatment after generating a mist using an atomization mechanism such as an ultrasonic transducer.

EP3496515 discloses a plasma nano mist generation device.

SUMMARY OF INVENTION

Technical Problem

However, in the electrostatic atomization method, it is necessary to form a closed circuit with the target side grounded to apply a high voltage, and there is a disadvantage that the target itself is included in the high voltage system.

In addition, the mist generation method using ultrasonic waves has a disadvantage that an atomization mechanism such as an ultrasonic transducer is required.

An object of the disclosure is to provide a plasma nano mist generation device capable of generating nano-sized mist with a simple configuration.

Solution to Problem

The invention is defined by the independent claim 1. A plasma nano mist generation device according to a first aspect includes: a discharge tube into which a solution is injected, the discharge tube being configured by a dielectric material; a liquid feeding unit configured to feed the solution into the discharge tube; an electrode provided in the discharge tube; a voltage application unit configured to apply a voltage to the electrode; a ground electrode provided on a side of a distal end of the electrode and on an outer periphery of the discharge tube, the ground electrode being grounded in the device itself; and a controller configured to control the liquid feeding unit and the voltage application unit such that the solution is fed to the discharge tube and a voltage is applied to the electrode in order to generate dielectric barrier discharge on the side of the distal end of the electrode to generate plasma nano mist.

In the plasma nano mist generation device according to the first aspect, the electrode may be a needle electrode or a cylindrical electrode.

In the plasma nano mist generation device according to the first aspect, the electrode may be the needle electrode.

The plasma nano mist generation device according to the first aspect may further includes an air blower that blows air into the discharge tube.

Further, in the plasma nano mist generation device according to the first aspect, the controller may control the voltage application unit to apply a voltage to the electrode, the voltage being set based on a correspondence relationship among a type of the solution, an outer diameter of the discharge tube, an outer diameter of the electrode, and a voltage applied to the electrode, the correspondence relationship being predetermined as generation-allowing conditions under which generation of the plasma nano mist is allowed.

Moreover, in the plasma nano mist generation device according to the first aspect, the controller may control the voltage application unit to apply a voltage to the electrode and control the liquid feeding unit to feed the solution at a feeding speed, the voltage and the feeding speed being set based on a correspondence relationship among a type of the solution, an outer diameter of the discharge tube, an outer diameter of the electrode, a voltage applied to the electrode, and the feeding speed of the solution, the correspondence relationship being predetermined as generation-allowing conditions under which generation of the plasma nano mist is allowed.

Advantageous Effects of Invention

According to the disclosure, an effect of generating nano-sized mist with a simple configuration is provided.

BRIEF DESCRIPTION OF DRAWINGS

Fig. 1A is a configuration diagram of a plasma nano mist generation device.
Fig. 1B is a view illustrating a streamer viewed from a lower side of a discharge tube.
Fig. 2 is an exploded view of a discharge unit.
Fig. 3 is a cross-sectional perspective view of the discharge unit.
Fig. 4 is a graph showing a correspondence relationship among an outer diameter of an electrode, an applied voltage, and a feeding speed of a solution when an outer diameter of a discharge tube is 8 mm, the graph also showing conditions under which plasma nano mist can be generated.
Fig. 5 is a graph showing a correspondence relationship among the outer diameter of the electrode, the applied voltage, and the feeding speed of the solution when the outer diameter of the discharge tube is 8 mm, the graph also showing suitable conditions among the conditions under which plasma nano mist can be generated.
Fig. 6A is a view showing nano mist generated by the plasma nano mist generation device.
Fig. 6B is a partially enlarged view of Fig. 6A.
Fig. 7 is a graph showing a correspondence relationship among the outer diameter of the electrode, the applied voltage, and the feeding speed of the solution when the outer diameter of the discharge tube is 10 mm, the graph also showing conditions under which plasma nano mist can be generated.
Fig. 8 is a graph showing a correspondence relationship among an outer diameter of an electrode, an applied voltage, and a feeding speed of a solution when an outer diameter of a discharge tube is 10 mm, the graph also showing suitable conditions among the conditions under which plasma nano mist can be generated.
Fig. 9 is a graph showing a correspondence relationship among the outer diameter of the electrode, the applied voltage, and the feeding speed of the solution when the outer diameter of the discharge tube is 15 mm, the graph also showing conditions under which plasma nano mist can be generated.
Fig. 10 is a graph showing a correspondence relationship among the outer diameter of the electrode, the applied voltage, and the feeding speed of the solution when the outer diameter of the discharge tube is 15 mm, the graph also showing suitable conditions among the conditions under which plasma nano mist can be generated.
Fig. 11 is a graph showing discharge characteristics for each of solutions and for each of feeding speeds of the solutions.
Fig. 12 is a graph showing a relationship between a feeding speed of ultra pure water and conductivity.
Fig. 13 is a graph showing a relationship between a feeding speed of phosphate buffered saline and conductivity.
Fig. 14 is a graph showing a relationship between an amount of generated hydrogen peroxide water and a feeding speed.
Fig. 15 is a graph showing a relationship between an amount of generated nitrite ion and a feeding speed.
Fig. 16 is a graph showing a relationship between an amount of generated nitrate ion and a feeding speed.
Fig. 17 is a configuration diagram of the plasma nano mist generation device including an air blower.
Fig. 18 is a graph showing a correspondence relationship among the outer diameter of the discharge tube, the outer diameter of the electrode, and the applied voltage when the solution is ultra pure water and phosphate buffered saline, the graph also showing conditions under which plasma nano mist can be generated.
Fig. 19 is a graph showing a correspondence relationship among the outer diameter of the discharge tube, the outer diameter of the electrode, and the applied voltage when the solution is ultra pure water and phosphate buffered saline, the graph also showing conditions under which plasma nano mist can be generated.
Fig. 20 is a table illustrating a gauge replacement table.

DESCRIPTION OF EMBODIMENTS

Hereinafter, an example of an embodiment of the disclosure will be described with reference to the drawings.

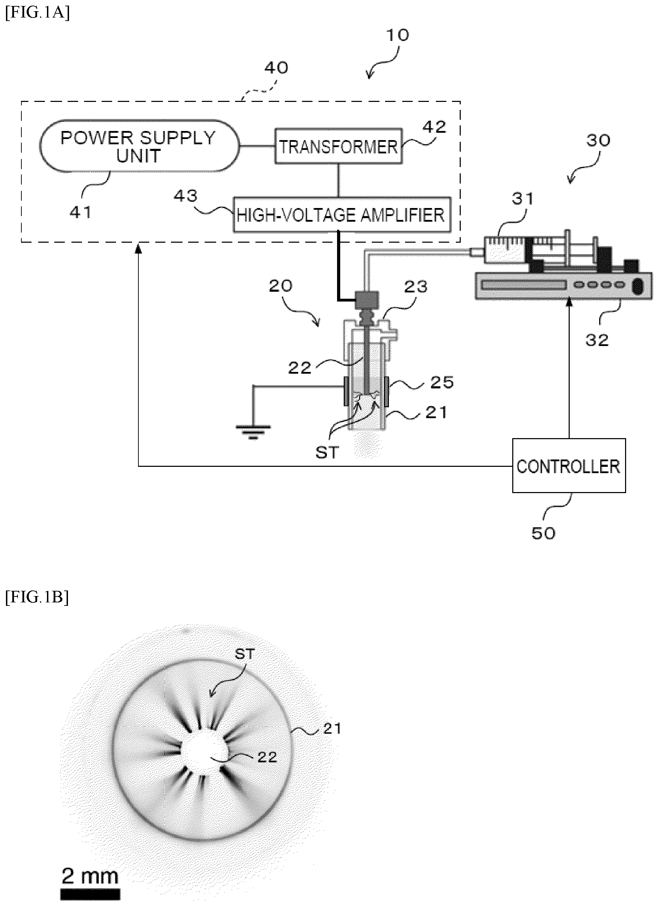

Fig. 1A shows a configuration diagram of a plasma nano mist generation device 10 according to the present embodiment. As shown in Fig. 1A, the plasma nano mist generation device 10 includes a discharge unit 20, a liquid feeding unit 30, a voltage application unit 40, and a controller 50.

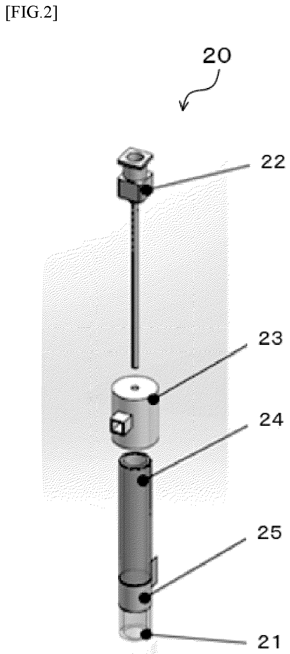

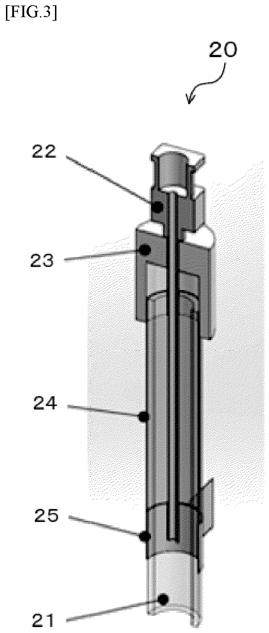

As also shown in Figs. 2 and 3, the discharge unit 20 includes a discharge tube 21 and an electrode 22. As the electrode 22, for example, a needle electrode or a cylindrical electrode is used. The needle electrode is, for example, an electrode having an outer diameter (diameter) of less than 2 mm, and the cylindrical electrode is, for example, an electrode having an outer diameter of 2 mm or more and 5 mm or less. The electrode 22 is configured by, for example, PLA resin (polylactic acid), and is inserted into a through hole of a socket 23 having the through hole. By fitting the socket 23 into the discharge tube 21, a side of a distal end of the electrode 22 is disposed in the discharge tube 21.

The discharge tube 21 is configured by, for example, a dielectric material such as borosilicate glass. An outer periphery of the discharge tube 21 is covered with a polyimide film tape 24.

Further, the outer periphery of the discharge tube 21 on the side of the distal end of the electrode 22 is covered with a ground electrode 25 configured by, for example, aluminum (Al). The ground electrode 25 is grounded in the plasma nano mist generation device 10. That is, since the ground electrode 25 is grounded in the own device to form a closed circuit, it is not necessary to ground on a side of a supply target of the plasma nano mist, and therefore the safety is higher than the case of using the electrostatic atomization method.

The other end of the electrode 22 is connected to a syringe pump 31 of the liquid feeding unit 30 described later. The electrode 22 has a hollow structure, and a solution is injected from the syringe pump 31.

The liquid feeding unit 30 feeds the solution into discharge tube 21. Specifically, the liquid feeding unit 30 includes the syringe pump 31 that delivers a solution and a drive unit 32 that drives the syringe pump 31.

The voltage application unit 40 applies a voltage to the other end of the electrode 22. The voltage application unit 40 includes a power supply unit 41, a transformer 42, and a high-voltage amplifier 43. The power supply unit 41 supplies an AC voltage of 100 V. The transformer 42 transforms the AC voltage supplied from the power supply unit 41 into a predetermined AC voltage. The high-voltage amplifier 43 amplifies the AC voltage supplied from the transformer 42 to a high voltage of several kV to several tens kV, for example, and applies the amplified AC voltage to the other end of the electrode 22. The frequency of the voltage applied to the other end of the electrode 22 is, for example, about 10 kHz.

The controller 50 controls the drive unit 32 of the liquid feeding unit 30 and the voltage application unit 40 such that the solution is fed to the discharge tube 21 and a voltage is applied to the other end of the electrode 22 in order to generate dielectric barrier discharge, more specifically, a corona-like dielectric barrier discharge on the side of the distal end of the electrode 22 to generate plasma nano mist. Here, the plasma nano mist refers to particles having a diameter due to plasma of less than 1 $\mu$m, that is, of nanometer sized.

The solution injected from the syringe pump 31 into the electrode 22 is charged when the solution passes through the electrode 22, and interacts with plasma generated at the distal end of the electrode 22 to generate the nano mist. A liquid delivery means other than a syringe pump may be used as long as a liquid can be delivered at a desired speed, and for example, a piezo micro pump or the like can be used in the case where a liquid is fed continuously.

Hereinafter, the principle of generation of the nano mist will be described. A streamer ST as shown in Figs. 1A and 1B is generated from the distal end of the electrode 22 toward the ground electrode 25. When an AC high voltage is applied to the electrode 22, the discharge tube 21 that is a dielectric material on the front surface of the electrode 22 and a layer of air between the electrode 22 and the discharge tube 21 are charged, and a reverse electric field is formed between the distal end of the electrode 22 where the charge locally increases and the surface of the discharge tube 21 at a position closest to the distal end of the electrode 22. Therefore, the streamer ST generated in a high electric field is limited, and a discharge space in which no spark is generated is obtained.

The streamer ST is a strongly ionized discharge path when discharge in air is started. When the electrode is disposed in the atmosphere and the voltage is greater than or equal to a certain value, the electrons gain energy from the electric field and travel toward the anode, during which an electronic avalanche is formed. Positive ions having a large mass generated by ionization are left behind the electronic avalanche, a strong electric field due to space charges is generated on the anode side which is the head of the electronic avalanche, and the electrons ionized by light emitted from the electronic avalanche are attracted. An inside of the streamer ST is plasma, and at a tip thereof, the positive ion cloud moves at a high speed in the direction of other electrodes. In addition, there is strong light emission from a tip of the positive ion cloud, and a photoionization region is formed in the vicinity of the streamer ST. Since the streamer ST is unstable in a discharge form represented by spark discharge, the streamer ST is stabilized by the dielectric barrier discharge.

In addition, when a liquid is delivered in the electrode 22, the liquid is charged by a high voltage, and atomized at the distal end of the electrode 22 by so-called Coulomb repulsion. The atomized droplets come into contact with the streamer ST, which is a strongly ionized discharge path, causing a further ionization state, and are highly miniaturized into nano mist.

Since the plasma nano mist generation device 10 has the above-described configuration, an atomization mechanism such as an ultrasonic transducer is not required, and plasma nano mist can be generated with a simple configuration.

Whether the plasma nano mist can be generated or not depends on the type of the solution, the outer diameter of the discharge tube 21, the outer diameter of the electrode 22, the voltage applied to the electrode 22, and the feeding speed of the solution.

Therefore, the controller 50 controls the voltage application unit 40 to apply a voltage to the electrode 22 and controls the drive unit 32 of the liquid feeding unit 30 to feed the solution at a feeding speed, the voltage and the feeding speed being set based on table data representing a correspondence relationship among the type of solution, the outer diameter of the discharge tube 21, the outer diameter of the electrode 22, the voltage applied to the electrode 22, and the feeding speed of the solution, the correspondence relationship being predetermined as generation-allowing conditions under which generation of the plasma nano mist is allowed. As a result, the plasma nano mist is generated. The generated plasma nano mist can be used in a wide range of applications such as medical care and cosmetics. In addition, since the generated plasma nano mist is charged, wettability is improved.

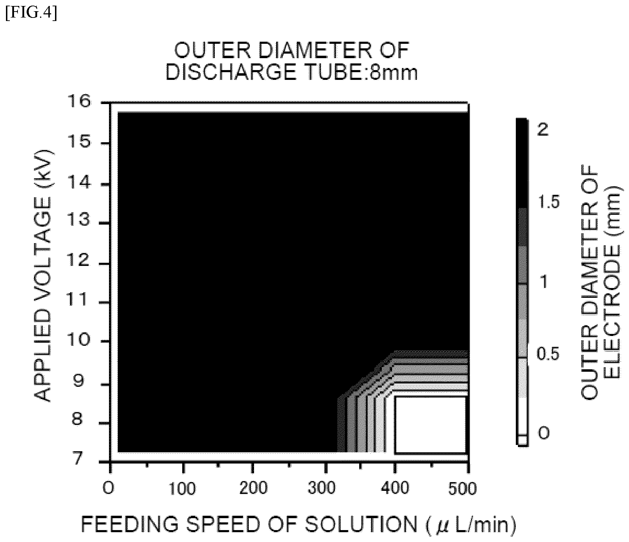

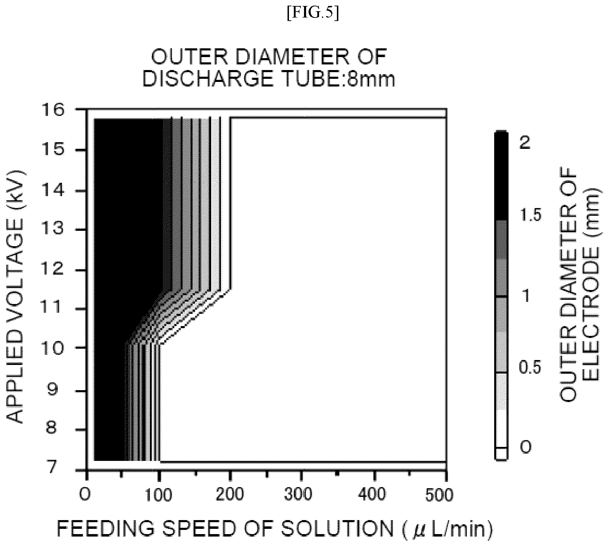

Fig. 4 shows a correspondence relationship among an applied voltage (kV) applied to the electrode 22, a feeding speed ($\mu$L/min) of a solution, and an outer diameter (diameter, mm) of the electrode 22 when the outer diameter (diameter, mm) of the discharge tube 21 is 8 mm. The solution is ultra pure water (UPW), and the solution in Figs. 5 to 10 described later is ultra pure water as well.

As shown in Fig. 4, assuming that the applied voltage is P (kV), the feeding speed ($\mu$L/min) of the solution is S, and the outer diameter (mm) of the electrode 22 is D, the plasma nano mist can be generated preferably under conditions that all the following Formulas (1) to (3) are satisfied.

$$7 \leq P \leq 17.5 \quad \text{... (1)}$$

$$0 < S < 500 \quad \text{... (2)}$$

$$0.25 \leq D \leq 5 \quad \text{... (3)}$$

Fig. 5 shows suitable conditions among the generation-allowing conditions for the plasma nano mist shown in Fig. 4. Here, the suitable conditions are conditions under which a phenomenon that droplets are generated from the discharge tube 21 does not occur. As illustrated in Fig. 5, a range of the suitable conditions is narrower than a range of the generation-allowing conditions shown in Fig. 4.

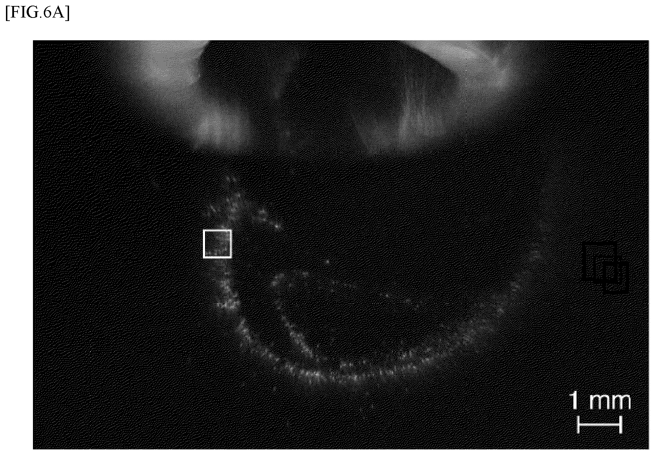

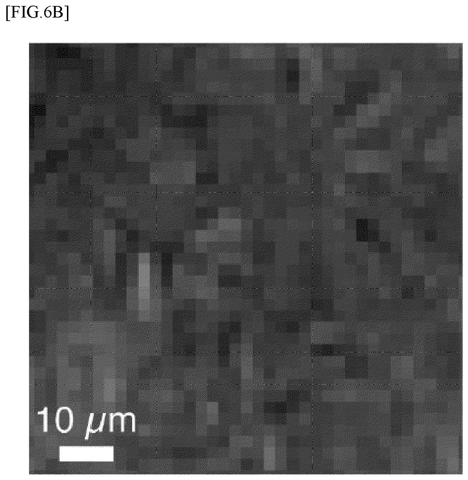

In Figs. 6A and 6B, the mist generated by the plasma nano mist generation device 10 using ultra pure water as the solution was photographed with a digital camera at an ISO sensitivity of 8000 and a shutter speed of 1/8000 seconds using a 10x magnification microscope lens. Fig. 6A is a photograph of the generated mist, and Fig. 6B is an enlarged view of a part of a range surrounded by a white frame of Fig. 6A. From Fig. 6B, it can be confirmed that the generated mist is equal to or smaller than a minimum pixel size (2150 nm×2150 nm) of the photographed camera, and is micro-sized to nano-sized, which is equal to or smaller than 2.15 $\mu$m.

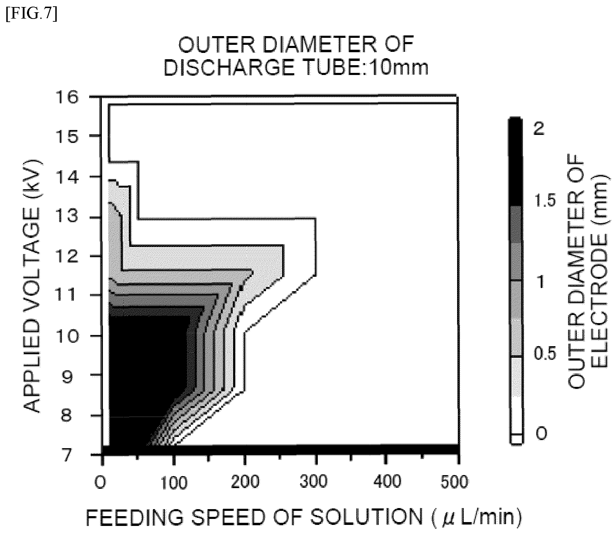

Fig. 7 shows a correspondence relationship among an applied voltage (kV) applied to the electrode 22, a feeding speed ($\mu$L/min) of the solution, and an outer diameter (diameter, mm) of the electrode 22 when the outer diameter (diameter, mm) of the discharge tube 21 is 10 mm.

As shown in Fig. 7, as compared with the case where the outer diameter (diameter, mm) of the discharge tube 21 is 8 mm in Fig. 4, the range of the generation-allowing conditions for the plasma nano mist is narrowed.

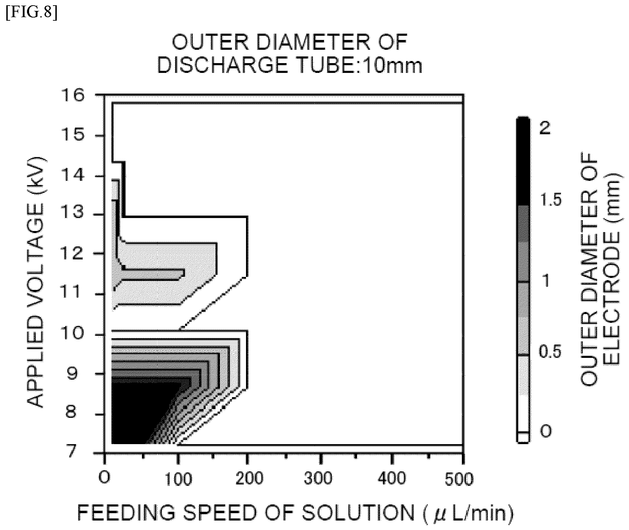

Fig. 8 shows suitable conditions among the generation-allowing conditions for the plasma nano mist shown in Fig. 7. As illustrated in Fig. 8, a range of the suitable conditions is narrower than a range of the generation-allowing conditions shown in Fig. 7.

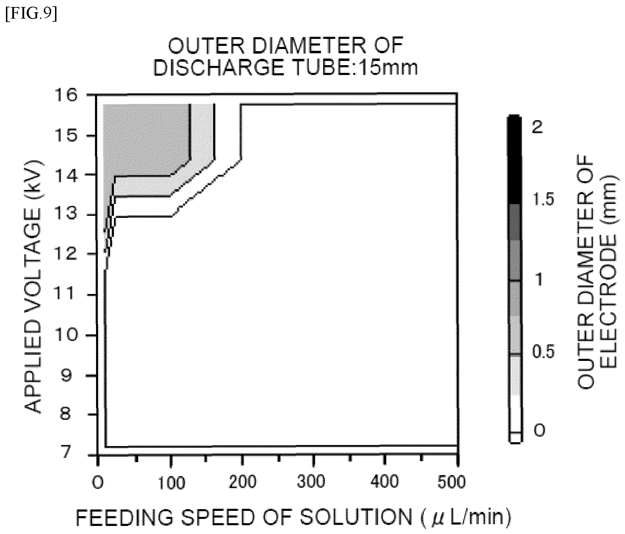

Fig. 9 shows a correspondence relationship among an applied voltage (kV) applied to the electrode 22, a feeding speed ($\mu$L/min) of the solution, and an outer diameter (diameter, mm) of the electrode 22 when the outer diameter (diameter, mm) of the discharge tube 21 is 15 mm.

As shown in Fig. 9, as compared with the case where the outer diameter (diameter, mm) of the discharge tube 21 is 8 mm in Fig. 4 and the case where the outer diameter (diameter, mm) of the discharge tube 21 is 10 mm in Fig. 7, the range of the generation-allowing conditions for the plasma nano mist is narrowed.

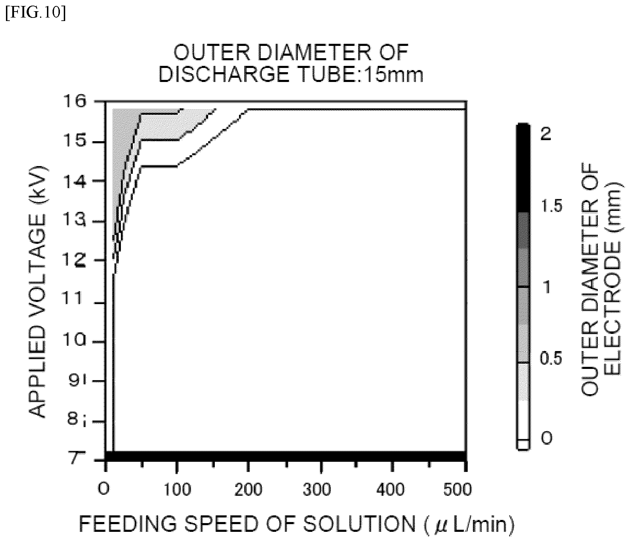

Fig. 10 shows suitable conditions among the generation-allowing conditions for the plasma nano mist shown in Fig. 9. As illustrated in Fig. 10, a range of the suitable conditions is narrower than a range of the generation-allowing conditions shown in Fig. 9.

Whether or not the solution easily become the mist depends on conductivity of the solution. That is, a solution having higher conductivity more easily become the mist, and a solution having lower conductivity less easily become the mist.

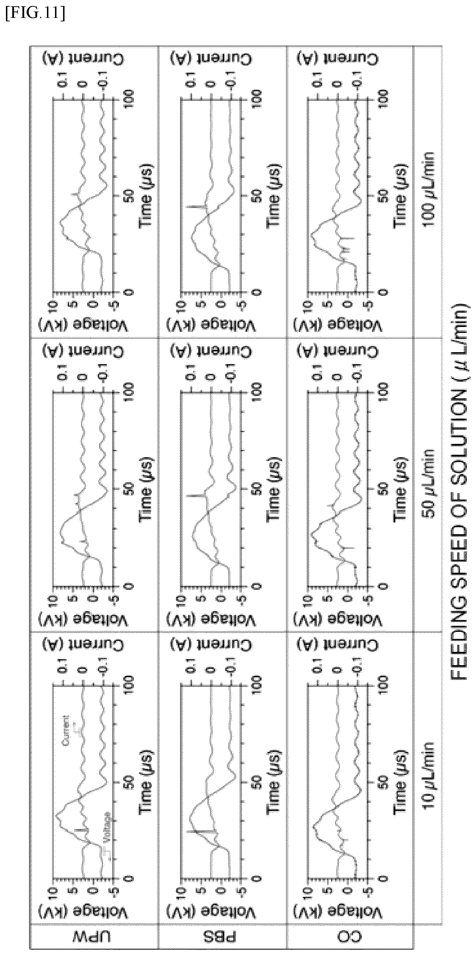

Fig. 11 shows results of measuring a voltage and a current applied to the electrode 22, that is, results of measuring discharge characteristics for three types of solutions: ultra pure water (UPW), phosphate buffered saline (PBS), and castor oil (CO: Castor Oil), at different feeding speeds of the solutions of 10 ($\mu$L/min), 50 ($\mu$L/min), and 100 ($\mu$L/min). As shown in Fig. 11, the discharge characteristics vary depending on the solution. The fact that the discharge characteristics of the solutions are different means that the conductivities of the solutions are different, and how easily the solutions become the mist is different.

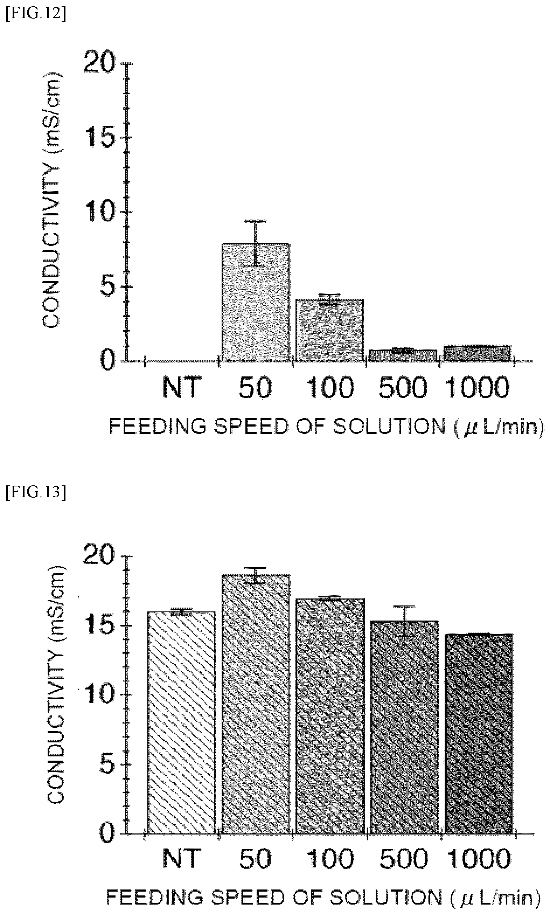

Fig. 12 shows results of measuring the conductivity of ultra pure water at different feeding speeds of ultra pure water of 50 ($\mu$L/min), 100 ($\mu$L/min), and 1000 ($\mu$L/min).

In addition, Fig. 13 shows results of measuring the conductivity of phosphate buffered saline at different feeding speeds of phosphate buffered saline of 50 ($\mu$L/min), 100 ($\mu$L/min), and 1000 ($\mu$L/min). As shown in Figs. 12 and 13, different types of solutions have different conductivities. That is, how easily the solutions become the mist is different.

Therefore, by controlling the voltage to be applied to the electrode 22 and the feeding speed of the solution according to the type of the solution, it is possible to appropriately generate the nano mist.

In addition, there is a case where reactive chemical species having a disinfection or sterilization effect such as hydrogen peroxide water ($H_2O_2$), nitrite ions ($NO_2^-$), and nitrate ions ($NO_3^-$) are generated by an interaction between the plasma generated at the distal end of the electrode 22 and the solution.

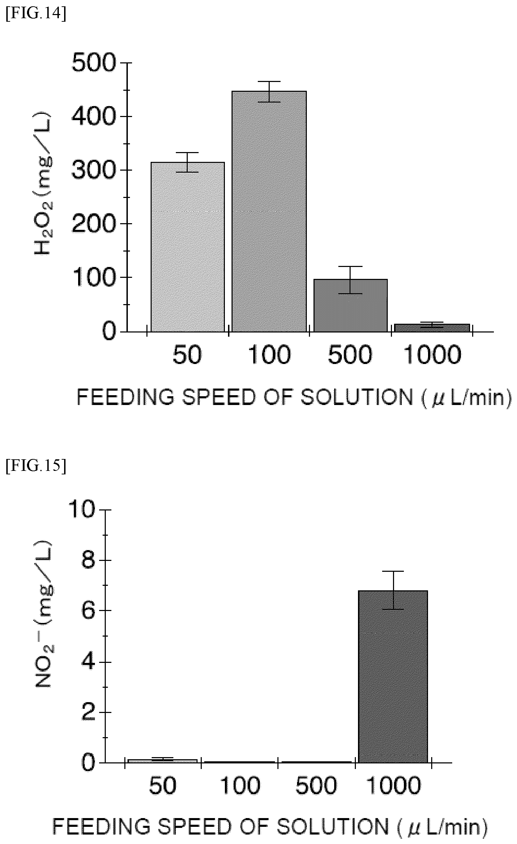

Fig. 14 shows results of measuring an amount (mg/L) of hydrogen peroxide water dissolved in ultra pure water (UPW) by changing the feeding speed ($\mu$L/min) of the solution when the solution is ultra pure water.

Fig. 15 shows results of measuring an amount (mg/L) of nitrite ions dissolved in ultra pure water (UPW) by changing the feeding speed ($\mu$L/min) of the solution when the solution is ultra pure water.

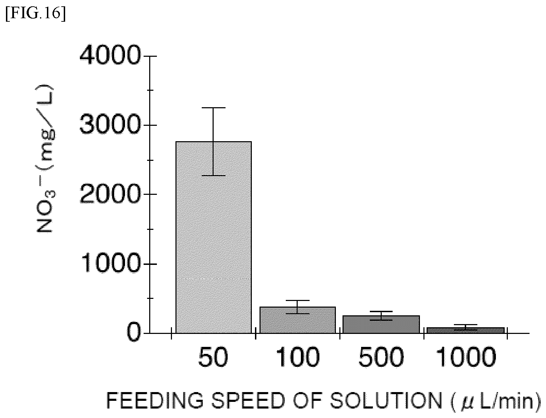

Fig. 16 shows results of measuring an amount (mg/L) of nitrate ions dissolved in ultra pure water (UPW) by changing the feeding speed ($\mu$L/min) of the solution when the solution is ultra pure water.

As shown in Figs. 14 to 16, it is found that the amount of the reactive chemical species varies depending on the type of the reactive chemical species. If it is desired to inhibit production of the reactive chemical species, a scavenger may be mixed into the solution.

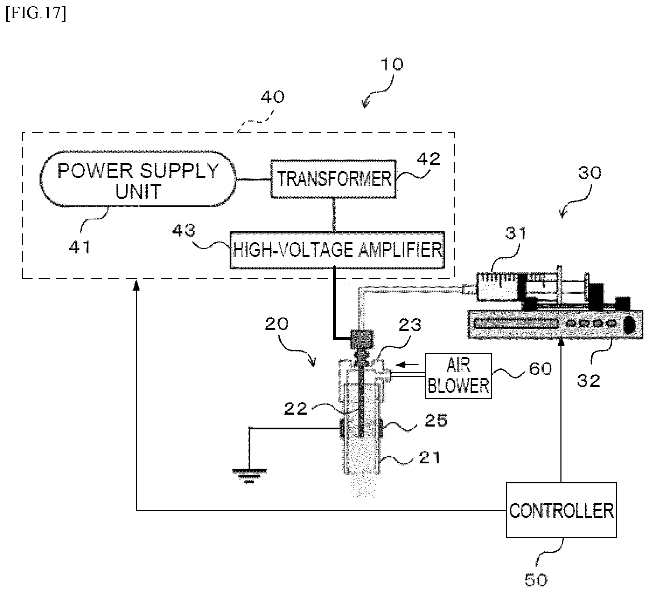

As illustrated in Fig. 17, an air blower 60 that blows air into the discharge tube 21 may be provided. As a result, the generated plasma nano mist is easily sent outside of the discharge tube 21.

In Fig. 1A, the discharge tube 21 is set to face downward, but the discharge tube 21 may face upward or sideways.

When it is desired to generate a large amount of nano mist, a bundle of a plurality of the discharge tubes 21 and the electrodes 22 can be used.

As illustrated in Fig. 17, in the case of the configuration in which air is blown into the discharge tube 21 and in the case where the direction of the discharge tube 21 is directed upward in the configuration illustrated in Fig. 1A, the feeding speed of the solution does not affect generation of droplets. In this case, the controller 50 controls the voltage application unit 40 to apply a voltage to the electrode 22, the voltage being set based on table data representing a correspondence relationship among the type of solution, the outer diameter of the discharge tube 21, the outer diameter of the electrode 22, and the voltage applied to the electrode 22, the table data representing the correspondence relationship predetermined as generation-allowing conditions under which generation of the plasma nano mist is allowed.

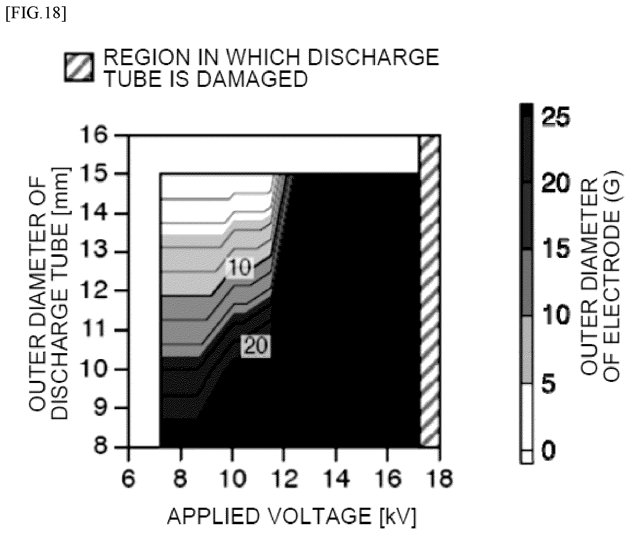

Fig. 18 shows a correspondence relationship among the outer diameter (mm) of the discharge tube 21, the outer diameter (G) of the electrode 22, and the applied voltage (kV) in ultra pure water and phosphate buffered saline as conditions under which plasma nano mist can be generated.

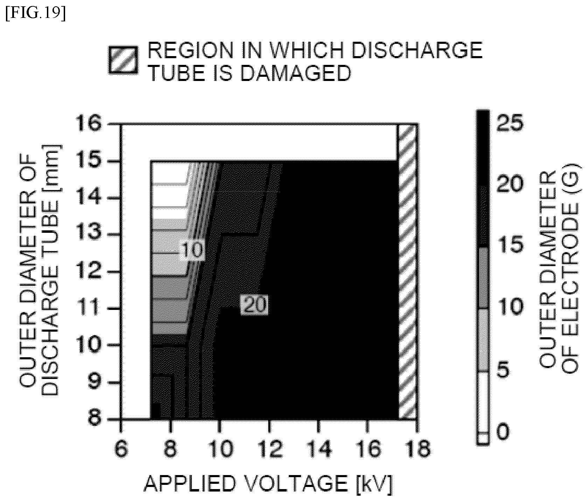

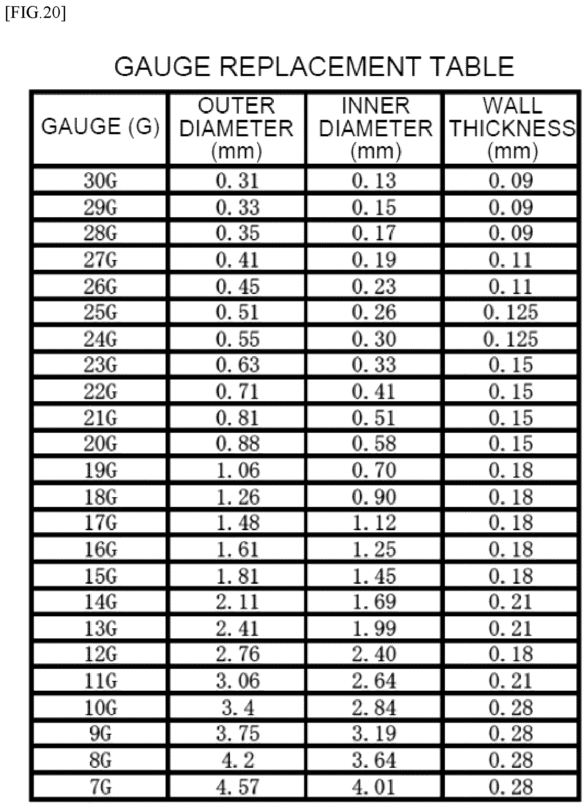

Fig. 19 shows a correspondence relationship among the outer diameter (mm) of the discharge tube 21, the outer diameter (G) of the electrode 22, and the applied voltage (kV) in castor oil. Fig. 20 shows a gauge replacement table representing a correspondence relationship among a gauge (G), an outer diameter (mm), an inner diameter (mm), and a wall thickness (mm).

In Figs. 18 and 19, a white region is a region where the plasma nano mist is not generated, and the other region is a region with conditions under which plasma nano mist can be generated. Further, since the discharge tube 21 is damaged at an applied voltage larger than 17.5 kV, it is necessary to set the applied voltage to 17.5 kV or less. As shown in Figs. 18 and 19, it is found that the conditions under which plasma nano mist can be generated are different between the case where the solution is ultra pure water and phosphate buffered saline and the case where the solution is castor oil.

Although the embodiment of the disclosure has been described in detail with reference to the drawings, the specific configuration is not limited to this embodiment, and includes design changes and the like without departing from the gist of the disclosure.

For example, the solution is not limited to ultra pure water, castor oil, and phosphate buffered saline, and methanol, ethanol, 1-propanol, 2-propanol, butanol, perfume (including both oily and aqueous), a pigment-based ink, a dye-based ink, and the like may be used, for example. Specific examples of the perfume include an essential oil volatile oily perfume, and a perfume dissolved in alcohol, carbon disulfide, petroleum ether, or fatty oil can be used as a component. As the pigment-based ink or the dye-based ink, an ink using a vegetable oil derivative, for example, as a main component can be used. Further, in the pigment-based ink, for example, it is possible to make the pigment-based ink dissolved in an organic solvent into the nano mist without any problem.

## Claims

1. A plasma nano mist generation device (10) comprising:

   a discharge tube (21) into which a solution is injected, the discharge tube being configured by a dielectric material;
   a liquid feeding unit (30) configured to feed the solution into the discharge tube;
   an electrode (22) provided in the discharge tube, the electrode being capable of delivering the solution;
   a voltage application unit (40) configured to apply a voltage to the electrode;
   a ground electrode (25) provided on a side of a distal end of the electrode and on an outer periphery of the discharge tube, the ground electrode being grounded in the device itself; and
   a controller (50) configured to control the liquid feeding unit and the voltage application unit such that the solution is fed to the discharge tube, and a voltage is applied to the electrode, in order to generate dielectric barrier discharge on the side of the distal end of the electrode to generate plasma nano mist.

2. The plasma nano mist generation device according to claim 1, wherein:
   the electrode (22) is a needle electrode or a cylindrical electrode.

3. The plasma nano mist generation device according to claim 2, wherein:
   the electrode (22) is the needle electrode.

4. The plasma nano mist generation device according to any one of claims 1 to 3, further comprising:
   an air blower (60) that is configured to blow air into the discharge tube.

5. The plasma nano mist generation device according to any one of claims 1 to 4, wherein:
   the controller (50) is configured to control the voltage application unit (40) to apply a voltage to the electrode, the voltage being set based on a correspondence relationship among a type of the solution, an outer diameter of the discharge tube, an outer diameter of the electrode, and a voltage applied to the electrode, the correspondence relationship being predetermined as generation-allowing conditions under which generation of the plasma nano mist is allowed.

6. The plasma nano mist generation device according to any one of claims 1 to 4, wherein:
   the controller (50) is configured to control the voltage application unit (40) to apply a voltage to the electrode and control the liquid feeding unit to feed the solution at a feeding speed, the voltage and the feeding speed being set based on a correspondence relationship among a type of the solution, an outer diameter of the discharge tube, an outer diameter of the electrode, a voltage applied to the electrode, and the feeding speed of the solution, the correspondence relationship being predetermined as generation-allowing conditions under which generation of the plasma nano mist is allowed.

7. The plasma nano mist generation device according to claim 6, wherein:
   the feeding speed is faster than 0 µL/min and slower than 500 µL/min.

8. The plasma nano mist generation device according to any one of claims 1 to 7, wherein:

   the solution is any one of ultra pure water, castor oil, and phosphate buffered saline,
   the outer diameter of the discharge tube (21) is 8 mm or more and 15 mm or less,
   the outer diameter of the electrode (22) is 0.25 mm or more and 5 mm or less, and
   the voltage is 7 kV or more and 17.5 kV or less.

## Patentansprüche

1. Plasmananonebelerzeugungsvorrichtung (10), umfassend:

   ein Auslassrohr (21), in das eine Lösung eingespritzt wird, wobei das Auslassrohr durch ein dielektrisches Material ausgebildet ist;
   eine Flüssigkeitseinspeiseeinheit (30), dazu ausgestaltet, die Lösung in das Auslassrohr einzuspeisen;
   eine Elektrode (22), bereitgestellt in dem Auslassrohr, wobei die Elektrode in der Lage ist, die Lösung zuzuführen;
   eine Spannungsanwendungseinheit (40), dazu ausgestaltet, an der Elektrode eine Spannung anzulegen;
   eine Erdelektrode (25), bereitgestellt auf einer Seite eines distalen Endes der Elektrode und auf einem Außenumfang des Entladerohrs, wobei die Erdelektrode in der Vorrichtung selbst geerdet ist; und
   eine Steuerung (50), dazu ausgestaltet, die Flüssigkeitseinspeiseeinheit und die Spannungsanwendungseinheit so zu steuern, dass die Lösung in das Entladerohr eingespeist wird und eine Spannung an die Elektrode angelegt wird, um auf der Seite des distalen Endes der Elektrode eine dielektrische Barriereentladung zu erzeugen, um Plasmananonebel zu erzeugen.

2. Plasmananonebelerzeugungsvorrichtung nach Anspruch 1, wobei:
die Elektrode (22) eine Nadelelektrode oder eine zylindrische Elektrode ist.

3. Plasmananonebelerzeugungsvorrichtung nach Anspruch 2, wobei:
die Elektrode (22) die Nadelelektrode ist.

4. Plasmananonebelerzeugungsvorrichtung nach einem der Ansprüche 1 bis 3, ferner umfassend:
ein Luftgebläse (60), dazu ausgestaltet, Luft in das Auslassrohr zu blasen.

5. Plasmananonebelerzeugungsvorrichtung nach einem der Ansprüche 1 bis 4, wobei:
die Steuerung (50) dazu ausgestaltet ist, dass sie die Spannungsanwendungseinheit (40) steuert, um eine Spannung an die Elektrode anzulegen, wobei die Spannung basierend auf einem Korrespondenzverhältnis zwischen einem Typ der Lösung, einem Außendurchmesser des Entladerohrs, einem Außendurchmesser der Elektrode und einer an die Elektrode angelegten Spannung eingestellt wird, wobei das Korrespondenzverhältnis als erzeugungserlaubende Bedingungen, unter denen die Erzeugung des Plasmananonebels zulässig ist, vorbestimmt ist.

6. Plasmananonebelerzeugungsvorrichtung nach einem der Ansprüche 1 bis 4, wobei:
die Steuerung (50) dazu ausgestaltet ist, die Spannungsanwendungseinheit (40) zu steuern, um eine Spannung an die Elektrode anzulegen, und die Flüssigkeitseinspeiseeinheit zu steuern, um die Lösung mit einer Einspeisegeschwindigkeit einzuspeisen, wobei die Spannung und die Einspeisegeschwindigkeit basierend auf einem Korrespondenzverhältnis zwischen einem Typ der Lösung, einem Außendurchmesser des Entladerohrs, einem Außendurchmesser der Elektrode, einer an die Elektrode angelegten Spannung und der Einspeisegeschwindigkeit der Lösung eingestellt sind, wobei das Korrespondenzverhältnis als erzeugungserlaubende Bedingungen, unter denen die Erzeugung des Plasmananonebels zulässig ist, vorbestimmt ist.

7. Plasmananonebelerzeugungsvorrichtung nach Anspruch 6, wobei:
die Einspeisegeschwindigkeit schneller als 0 µl/min und langsamer als 500 µl/min ist.

8. Plasmananonebelerzeugungsvorrichtung nach einem der Ansprüche 1 bis 7, wobei:

die Lösung aus einem jeglichen von Reinstwasser, Rizinusöl und phosphatgepufferter Kochsalzlösung besteht,
der Außendurchmesser des Auslassrohrs (21) 8 mm oder mehr und 15 mm oder weniger beträgt,
der Außendurchmesser der Elektrode (22) 0,25 mm oder mehr und 5 mm oder weniger beträgt, und
die Spannung 7 kV oder mehr und 17,5 kV oder weniger beträgt.

## Revendications

1. Un dispositif de génération de nano-brouillard de plasma (10) comprenant :

un tube à décharge (21) dans lequel une solution est injectée, le tube à décharge étant constitué par un matériau diélectrique ;
une unité d'alimentation en liquide (30) configurée pour amener la solution dans le tube à décharge ;
une électrode (22) disposée dans le tube à décharge, l'électrode étant capable de délivrer la solution ;
une unité d'application de tension (40) configurée pour appliquer une tension à l'électrode ;
une électrode de mise à la terre (25) disposée sur un côté d'une extrémité distale de l'électrode et sur une périphérie extérieure du tube à décharge, l'électrode de mise à la terre étant mise à la terre dans le dispositif lui-même ; et
un organe de contrôle (50) constitué pour contrôler l'unité d'alimentation en liquide et l'unité d'application de tension de telle sorte que la solution soit alimentée au tube de décharge, et une tension est appliquée à l'électrode, afin de générer une décharge à barrière diélectrique sur le côté de l'extrémité distale de l'électrode aux fins de générer un nano-brouillard de plasma.

2. Le dispositif de génération de nano-brouillard de plasma selon la revendication 1, dans lequel :
l'électrode (22) est une électrode de type à aiguille ou une électrode de type cylindrique.

3. Le dispositif de génération de nano-brouillard de plasma selon la revendication 2, dans lequel :
l'électrode (22) est l'électrode de type à aiguille.

4. Le dispositif de génération de nano-brouillard de plasma selon l'une quelconque des revendications 1 à 3, comprenant en outre :
un ventilateur d'air (60) configuré pour souffler de l'air dans le tube à décharge.

5. Le dispositif de génération de nano-brouillard de plasma selon l'une quelconque des revendications 1 à 4, dans lequel :

l'organe de contrôle (50) est configuré pour contrôler l'unité d'application de tension (40) aux fins d'appliquer une tension à l'électrode, la tension étant réglée sur la base d'une relation de correspondance avec soit un type de solution, soit un diamètre extérieur du tube à décharge, soit un diamètre extérieur de l'électrode, soit une tension appliquée à l'électrode, la relation de correspondance étant prédéterminée en tant que condition permettant la génération dans laquelle la génération du nano-brouillard de plasma est autorisée.

6. Le dispositif de génération de nano-brouillard de plasma selon l'une quelconque des revendications 1 à 4, dans lequel :

l'organe de contrôle (50) est configuré pour contrôler l'unité d'application de tension (40) aux fins d'appliquer une tension à l'électrode et contrôler l'unité d'alimentation en liquide pour amener la solution à une vitesse d'alimentation, la tension et la vitesse d'alimentation étant réglées sur la base d'une relation de correspondance entre un type de solution, un diamètre extérieur du tube à décharge, un diamètre extérieur de l'électrode, une tension appliquée à l'électrode et la vitesse d'alimentation de la solution, la relation de correspondance étant prédéterminée en tant que condition permettant la génération dans laquelle la génération du nano-brouillard de plasma est autorisée.

7. Le dispositif de génération de nano-brouillard de plasma selon la revendication 6, dans lequel :
la vitesse d'alimentation est supérieure à 0 $\mu$L/min et inférieure à 500 $\mu$L/min.

8. Le dispositif de génération de nano-brouillard de plasma selon l'une quelconque des revendications 1 à 7, dans lequel :

la solution est l'une des solutions suivantes : eau ultra pure, huile de ricin et solution saline tamponnée au phosphate,
le diamètre extérieur du tube à décharge (21) est de 8 mm ou plus et de 15 mm ou moins,
le diamètre extérieur de l'électrode (22) est de 0,25 mm ou plus et de 5 mm ou moins, et
la tension est de 7 kV ou plus et de 17,5 kV ou moins.

[FIG.1A]

[FIG.1B]

2 mm

[FIG.2]

[FIG.3]

[FIG.4]

OUTER DIAMETER OF
DISCHARGE TUBE:8mm

[FIG.5]

OUTER DIAMETER OF
DISCHARGE TUBE:8mm

[FIG.6A]

[FIG.6B]

10 μm

[FIG.7]

OUTER DIAMETER OF
DISCHARGE TUBE:10mm

[FIG.8]

OUTER DIAMETER OF
DISCHARGE TUBE:10mm

[FIG.9]

OUTER DIAMETER OF
DISCHARGE TUBE:15mm

[FIG.10]

OUTER DIAMETER OF DISCHARGE TUBE:15mm

[FIG.11]

[FIG.12]

[FIG.13]

[FIG.14]

[FIG.15]

[FIG.16]

[FIG.17]

[FIG.18]

REGION IN WHICH DISCHARGE
TUBE IS DAMAGED

[FIG.19]

REGION IN WHICH DISCHARGE TUBE IS DAMAGED

[FIG.20]

## GAUGE REPLACEMENT TABLE

| GAUGE (G) | OUTER DIAMETER (mm) | INNER DIAMETER (mm) | WALL THICKNESS (mm) |
|---|---|---|---|
| 30G | 0.31 | 0.13 | 0.09 |
| 29G | 0.33 | 0.15 | 0.09 |
| 28G | 0.35 | 0.17 | 0.09 |
| 27G | 0.41 | 0.19 | 0.11 |
| 26G | 0.45 | 0.23 | 0.11 |
| 25G | 0.51 | 0.26 | 0.125 |
| 24G | 0.55 | 0.30 | 0.125 |
| 23G | 0.63 | 0.33 | 0.15 |
| 22G | 0.71 | 0.41 | 0.15 |
| 21G | 0.81 | 0.51 | 0.15 |
| 20G | 0.88 | 0.58 | 0.15 |
| 19G | 1.06 | 0.70 | 0.18 |
| 18G | 1.26 | 0.90 | 0.18 |
| 17G | 1.48 | 1.12 | 0.18 |
| 16G | 1.61 | 1.25 | 0.18 |
| 15G | 1.81 | 1.45 | 0.18 |
| 14G | 2.11 | 1.69 | 0.21 |
| 13G | 2.41 | 1.99 | 0.21 |
| 12G | 2.76 | 2.40 | 0.18 |
| 11G | 3.06 | 2.64 | 0.21 |
| 10G | 3.4 | 2.84 | 0.28 |
| 9G | 3.75 | 3.19 | 0.28 |
| 8G | 4.2 | 3.64 | 0.28 |
| 7G | 4.57 | 4.01 | 0.28 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011071182 A
- JP 2019058862 A
- EP 3496515 A

**Non-patent literature cited in the description**

- **MASAAKI NAGATSU** ; **ENBO YANG** ; **HAN CHO** ; **AKIKAZU SAKUDO** ; **KAZUYOSHI IKUTA**. Bio- and Medical-Application of Plasma-Functionalized Nanoparticles. *Journal of the Surface Science Society of Japan*, 2013, vol. 34 (10), 535-540

- **MICHAEL, P.** ; **LAM, Y.T.** ; **FILIPE, E.C. et al.** Plasma polymerized nanoparticles effectively deliver dual siRNA and drug therapy in vivo. *Sci Rep*, 2020, vol. 10, 12836, https://doi.org/10.1038/s41598-020-69591-x